# Europäisches Patentamt
## European Patent Office
## Office européen des brevets

(19)

(11) Veröffentlichungsnummer: **0 173 860 B1**

(12) **EUROPÄISCHE PATENTSCHRIFT**

(45) Veröffentlichungstag der Patentschrift:
**20.04.88**

(51) Int. Cl.⁴: **C 04 B 35/52,** H 01 G 9/04, C 01 B 31/02

(21) Anmeldenummer: **85109615.6**

(22) Anmeldetag: **31.07.85**

(54) **Verfahren zur Herstellung von Glaskohlenstoff-Elektroden.**

(30) Priorität: **13.08.84 DE 3429768**

(43) Veröffentlichungstag der Anmeldung:
**12.03.86 Patentblatt 86/11**

(45) Bekanntmachung des Hinweises auf die Patenterteilung:
**20.04.88 Patentblatt 88/16**

(84) Benannte Vertragsstaaten:
**DE FR GB IT NL SE**

(56) Entgegenhaltungen:
**EP - A - 0 036 601**
**DE - A - 1 694 909**
**DE - A - 2 204 749**
**DE - A - 2 418 507**
**DE - A - 2 623 828**

**CHEMICAL ABSTRACTS, Band 85, Nr. 22, November 1976, Seite 150, Nr. 162632q, Columbus, Ohio, US; & JP - A - 74 111 890 (TOKAI ELECTRODE MFG. CO., LTD.) 24.10.1974**
**PATENT ABSTRACTS OF JAPAN, Band 2, Nr. 24, 16. Februari 1978, Seite 4071, C77; & JP - A - 52 120 294 (HITACHI SEISAKUSHO K.K.) 08.10.1977**
**JAPANESE PATENTS GAZETTE, 25. April 1984, Seite 17, Derwent Publications LTD., Section Chemical, Week 8411; JP - A - 59 021 512 (MITSUBISHI PENCIL)**

(73) Patentinhaber: **Siemens Aktiengesellschaft Berlin und München, Wittelsbacherplatz 2, D-8000 München 2 (DE)**

(72) Erfinder: **Edeling, Martin, Dr., Barkhovenallee 22, D-4300 Essen 16 (DE)**

## Beschreibung

Die Erfindung betrifft ein Verfahren zur Herstellung von Glaskohlenstoff-Ekektroden durch Härtung und Pyrolyse von duroplastischen carbonisierbaren Harzen sowie die Verwendung der nach diesem Verfahren hergestellten Glaskohlenstoff-Elekroden.

Glaskohlenstoff has sich als Elektrodenmaterial auf elektrochemischem Gebiet vielfach bewärt. Besonders in aktivierter Form ist Glaskohlenstoff, durch die bei der Aktivierung erreichbare grosse Oberfläche (Rauhigkeitsfaktor $> 10^4$), von speziellem Interesse. Beispiele hierfür sind die elektrochemische Energiespeicherung (DE-A-3 011 701) und Anwendungen in der Elektromedizin (DE-A-2 613 072), d.h. die Verwendung von aktivierten Glaskohlenstoff-Elektroden in elektrochemischen Doppelschichtkondensatoren bzw. als Reizelektroden, insbesondere in Herzschrittmachern.

Bei vielen Anwendungen werden dünne Elektroden, insbesondere in Scheibenform, mit einer Dicke von weniger als 1 mm benötigt. Als Ausgangsmaterial hierzu eignen sich Folien oder Filme aus nichtschmelzbaren, insbesondere vernetzten Kunststoffen, die durch Pyrolyse in Glaskonhlenstoff übergeführt werden können (DE-A-30 11 702). Geeignete vernetzte Kunststoffe, die bei der Pyrolyse einen hohen Kohlenstoffanteil hinterlassen und somit eine gute Kohlenstoffausbeute ergeben, sind Phenol-Formaldehydharze und Furanharze.

Bei der Herstellung dünner Folien bzw. Filme aus Kunststoffen der vorstehend genannten Art ergeben sich jedoch eine Reihe von Schwierigkeiten. So dürfen die hydrophilen Harze die Giessform nicht benetzen, um nach der Vernetzung des Harzes eine einwandfreie Trennung zu gewährleisten. Ferner verhindert die grosse Oberflächenspannung eine gleichmässig dünne Verteilung des Harzes auf einer hydrophoben Unterlage. Die Verwendung von Lösungsmitteln, wie es bei der Herstellung von Folien aus nicht-extrudierbaren Kunststoffen der Fall ist, führt hinwiederum zu einer Verteuerung der Endprodukte und beinhaltet darüber hinaus die Gefahr einer Porenbildung.

Zur Herstellung von Glaskohlenstoff-Elektroden kann auch in der Weise vorgegangen werden, dass das Harz in einen Spalt, der durch zwei in einem entsprechenden Abstand voneinander fixierten ebenen Platten gebildet wird, gegossen und darin vernetzt wird. Aus den nach der Härtung erhaltenen Scheiben werden dann Elektroden-Rohlinge ausgestanzt und pyrolysiert. Eine derartige Verfahrensweise ist aber insofern aufwendig, als sie jeweils nur die Fertigung geringer Stückzahlen erlaubt, d.h. es ist keine kontinuierliche Fertigung möglich. Darüber hinaus können auf diese Weise auch keine dünnen Elektroden hergestellt werden.

Aufgabe der Erfindung ist es, das Verfahren der eingangs genannten Art zur Herstellung von Glaskohlenstoff-Elektroden aus duroplastischen carbonisierbaren Harzen derart auszugestalten, dass in kostengünstiger Weise eine kontinuierliche Herstellung von dünnen Elektroden mit gleichbleibender Dicke ermöglicht wird.

Dies wird erfindungsgemäss dadurch erreicht, dass dem Harz 1 bis 10 Masse-% eines pyrolysierbaren organischen Fettalkohol- oder Fettsäurederivates der allgemeinen Formel RCOOR' mit mindestens 14 Kohlenstoffatomen zugesetzt werden und dass das Harz in dünner Schicht auf einer hydrophoben Unterlage ausgebreitet und anschliessend gehärtet und pyrolysiert wird.

Mittels des erfindungsgemässen Verfahrens lassen sich kontinuierlich dünne Glaskohlenstoff-Elektroden herstellen, die eine gleichbleibende Dicke — in der gewünschten Stärke — aufweisen, d.h. diese Elektroden erfüllen die geforderte hohe Präzision der geometrischen Abmessungen, insbesondere hinsichtlich Welligkeit und Planheit.

Zu diesem Zweck dienen insbesondere die den Harzen zugesetzten speziellen organischen Verbindungen. Diese Verbindungen sind oberflächenaktiv, d.h. sie reduzieren die Oberflächenspannung des Harzes und ermöglichen somit, dass sich das Harz in dünner Schicht gleichmässig auf der hydrophoben Unterlage, die vorzugsweise aus Kunststoff besteht, ausbreitet. Die oberflächenaktiven Substanzen werden dem Harz in einer Menge zwischen 1 und 10 Masse-% zugesetzt; die jeweilige Menge richtet sich dabei nach der gewünschten Dicke des Harzfilms. Vorzugsweise gelangen 4 bis 5 Masse-% zum Einsatz.

Die den Harzen zugesetzten Verbindungen, die auch in Form von Gemischen verwendet werden können, sind Fettalkohol- bzw. Fettsäurederivate der Form RCOOR', welche wenigstens 14 C-Atome aufweisen. Unter Fettalkoholen (R'OH) werden dabei primäre höhere Alkohole verstanden, wie Laurinalkohol ($C_{12}H_{25}OH$), Myristinalkohol ($C_{14}H_{29}OH$), Cetylalkohl ($C_{16}H_{33}OH$) und Stearinalkohol ($C_{18}H_{37}OH$). Fettsäuren (RCOOH) sind aliphatische, gesättigte oder ungesättigte Carbonsäuren, wie Laurinsäure ($C_{11}H_{23}COOH$), Myristinsäure ($C_{13}H_{27}COOH$), Palmitinsäure ($C_{15}H_{31}COOH$), Stearinsäure ($C_{17}H_{35}COOH$) und Ölsäure ($C_{17}H_{33}COOH$).

Beim erfindungsgemässen Verfahren gelangen nun folgende Verbindungstypen RCOOR' zum Einsatz:

– Fettalkoholderivate mit einem kurzen aliphatischen Rest R, der auch ungesättigter Natur sein kann; vorzugsweise wird hierbei Stearylmethacrylat ($C_3H_5COOC_{18}H_{37}$) verwendet.

– Fettsäurederivate mit einem kurzen aliphatischen Rest R', insbesondere Ethyllaureat ($C_{11}H_{23}COOC_2H_5$), sowie Ethylpalmitat ($C_{15}H_{31}COOC_2H_5$) und Butylstearat ($C_{17}H_{35}COOC_4H_9$).

– Fettsäuren, d.h. Verbindungen mit R' = H; vorzugsweise wird hierbei Ölsäure ($C_{17}H_{33}COOH$) eingesetzt.

Die Verbindungen der vorstehend genannten Art müssen pyrolysierbar sein, sie dürfen ausserdem die elektrochemischen Eigenschaften des Glaskohlenstoffs nicht wesentlich beeinträchtigen. Bei der Herstellung der Glaskohlenstoff-Elektroden ist es vorteilhaft, die Polarität dieser Verbindungen auf das jeweilige Harz abzustimmen.

Beim erfindungsgemässen Verfahren wird als carbonisierbares Harz vorzugsweise Furfurylalkohol oder ein Phenol-Furanharz eingesetzt. Es können aber auch andere duroplastische carbonisierbare Harze verwendet werden, beispielsweise Phenol-Formaldehydharze.

Die Härtung von duroplastischen Harzen bzw. Kunststoffen kann mit basischen oder sauren Verbindungen erfolgen. Beim erfindungsgemässen Verfahren werden als Härter im allgemeinen starke Säuren eingesetzt, vorzugsweise wird p-Toluolsulfonsäure verwendet. Die Härtung selbst erfolgt bevorzugt bei Temperaturen von 150 bis 200°C. Dauer der Härtung: etwa 1 bis 5 h.

Die Pyrolyse erfolgt vorzugsweise bei Temperaturen bis zu 1100°C. Bei der Pyrolyse wird der duroplastische Kunststoff carbonisiert und dabei in Glaskohlenstoff übergeführt. Gleichzeitig erfolgt auch eine Pyrolyse der dem Harz zugesetzten organischen Substanz. Die Pyrolysedauer beträgt im allgemeinen etwa 12 bis 36 h (einschliesslich Aufheiz- und Abkühlzeit), vorzugsweise ca. 12 h. Die Endtemperatur wird dabei für etwa 1 bis 5 h gehalten.

Bei der Herstellung der Glaskohlenstoff-Elektroden nach dem erfindungsgemässen Verfahren kann beispielsweise folgendermassen vorgegangen werden. Dem Harz wird die erforderliche Menge an Härter sowie die oberflächenaktive Substanz zugesetzt, dann wird entgast und das Gemisch anschliessend in dünner Schicht auf eine hydrophobe Folie aus Kunststoff gegossen und auf eine Temperatur von etwa 60 bis 80°C erwärmt; die Trägerfolie besteht dabei vorzugsweise aus Polyethylen, Polytetrafluorethylen oder Polypropylen. Nach ca. 5 bis 20 min wird eine flexible, selbsttragende Rohfolie erhalten, deren Oberfläche — aufgrund des Zusatzes der organischen Verbindung — mit einem dünnen Ölfilm bedeckt ist. Dieser Ölfilm bewirkt bei Raumtemperatur zusätzlich einen Antihafteffekt und gestattet somit beispielsweise bei einem kontinuierlichen Verfahren ein Aufwickeln des Rohmaterials, d.h. der Rohfolie.

Aus der Rohfolie werden die Elektroden-Rohlinge in der gewünschten Form ausgestanzt und an der Luft bei Temperaturen zwischen 150 und 200°C, vorteilhafterweise gestapelt zwischen Graphitscheiben, gehärtet. Die nachfolgende Pyrolyse (in einer Inertgasatmosphäre) erfolgt ebenfalls vorteilhaft zwischen Graphitscheiben. Auf diese Weise wird nämlich die Planheit der Elektroden gewährleistet. Bei der Fertigung der Elektroden-Rohlinge ist im übrigen zu berücksichtigen, dass das Polymerisat während der Pyrolyse linear um 20% schrumpft.

Beim erfindungsgemässen Verfahren kann die Herstellung der Rohfolien vorteilhaft auch in zwei Verfahrensschritten erfolgen, d.h. die Polymerisation der Harze erfolgt in zwei Stufen. Dabei wird in einer ersten Stufe ein Harz-Vorpolymerisat hergestellt, indem die Ausgangsstoffe zunächst nur zu einem mehr oder weniger viskosen Harz vorkondensiert werden. Ein derartiges Harz-Vorpolymerisat ist bei Raumtemperatur für einige Zeit, d.h. bis zu mehreren Tagen, lagerfähig. In einer zweiten Stufe wird das Harz-Vorpolymerisat dann, unter Zusatz weiterer Härters und unter Wärmezufuhr, vollständing polymerisiert, d.h. in die Rohfolie übergeführt. Die oberflächenaktive Substanz wird bei diesem zweistufigen Verfahren im allgemeinen erst in der zweiten Stufe zugegeben, d.h. dem Harz-Vorpolymerisat zugesetzt; sie kann aber auch bereits dem Ausgangsgemisch beigefügt werden. Die aus den erhaltenen Rohfolien gefertigen Elektroden-Rohlinge werden dann in der beschriebenen Weise gehärtet und pyrolysiert.

Die bei der Pyrolyse erhaltenen Glaskohlenstoff-Elektroden können vorteilhaft noch aktiviert werden. Dabei wird auf den Elektroden oberflächlich eine mikroporöse Struktur mit Poren im nm-Bereich ($10^{-9}$ m und darunter) erzeugt. Durch die Aktivierung, die vorzugsweise durch Tempern an der Luft bei Temperaturen zwischen 450 und 500°C erfolgt (Dauer: ca. 3 bis 5h), kann die Doppelschichtkapazität der Glaskohlenstoff-Elektroden noch weiter erhöht werden.

Neben der Anwendung in elektrochemischen Doppelschichtkondensatoren können die nach dem erfindungsgemässen Verfahren hergestellten Glaskohlenstoff-Elektroden beispielsweise auch in der Elektromedizin verwendet werden.

Anhand von Ausführungsbeispielen soll die Erfindung noch näher erläutert werden.

*1. Herstellung eines Harz-Vorpolymerisats*
150 ml Furfurylalkohol werden bei ca. 50°C mit 5 ml einer 1%igen Lösung von p-Toluolsulfonsäure in Ethanol versetzt und unter Rühren innerhalb von 2 h auf eine Temperatur von ca. 90°C erwärmt. Das Reaktionsgemisch wird 2 h auf dieser Temperatur gehalten, wobei Kondensationswasser und geringe Mengen an Furfurylalkohol abdestillieren; anschliessend wird auf Raumtemperatur abgekühlt. Das auf diese Weise erhaltene Harz-Vorpolymerisat hat eine Viskosität von 2,5 Pa.s (bei 22°C) und ist bei Raumtemperatur mehrere Tage lang lagerfähig.

*2. Herstellung einer Folie*
20 g des Harz-Vorpolymerisats werden bei Raumtemperatur mit 0,5 ml einer 20%igen Lösung von p-Toluolsulfonsäure in Ethanol sowie mit 1 ml Stearylmethacrylat (4,3 Masse-%) versetzt. Es wird 5 min gerührt und anschliessend 15 min im Ölpumpenvakuum entgast. Dabei werden Reste von Lösungsmittel, Wasser und Furfurylalkohol, welche beim nachfolgenden Giessen der Folie zu einer Blasenbildung führen könnten, entfernt. Das Gemisch wird dann auf eine Polypropylenfolie (Dicke: 30 μm), welche auf eine auf 75°C erwärmte Metallplatte aufgespannt ist, gegossen und mit einer Rakel in einer Schichtdicke von 0,5 mm verteilt; bei einer kontinuierlichen Arbeitsweise könnte das Gemisch mittels eines Ziehschuhs auf die Folie aufgetragen werden. Das Harz darf die Trägerfolie nicht benetzen, damit diese nach der Polymerisation leicht entfernt werden kann. Nach etwa 3 bis 5 min ist der Gelpunkt des Harzes erreicht, und nach ca. 10 min wird die Trägerfolie von der Metallplatte abgehoben und auf Raumtemperatur abgekühlt und dann wird die flexible, selbsttragende Folie aus polymerisiertem Furfurylalkohol von der Trägerfolie abgezogen.

*3. Herstellung einer Glaskohlenstoff-Elektrode*
Aus der flexiblen Folie werden Elektroden-Rohlinge in der gewünschten Form ausgestanzt. Diese Rohlinge werden zwischen Graphitscheiben gestapelt und an der Luft 1 h bei ca. 180°C getempert, d.h. gehärtet. Die Graphitscheiben verhindern dabei ein Verziehen der Elektroden-Rohlinge sowie ein Zusammensintern beim Härten. Die gehärteten Rohlinge können, falls erforderlich, vor der nachfolgenden Pyrolyse mechanisch bearbeitet werden. Zur Pyrolyse werden die Rohlinge erneut zwischen Graphitscheiben gestapelt und dann innerhalb von 12 h bei Temperaturen bis zu 1100°C unter Inertgas (Stickstoff oder Argon) pyrolysiert. Auch hierbei dienen die Graphitscheiben dazu, die Planheit der Elektroden zu gewährleisten. Darüber hinaus ermöglicht die Porosität des Materials ein rasches Entweichen der Pyrolysegase trotz der dichten Stapelung. Bei der Pyrolyse, bei der die Endtemperatur für 1 h gehalten wird, schwinden die (gehärteten) Rohlinge linear um ca. 20% und die Dichte nimmt von 1,26 g . cm$^{-3}$ auf 1,51 g . cm$^{-3}$ zu. Die in der vorstehend beschriebenen Weise hergestellten Glaskohlenstoff-Elektroden können noch aktiviert werden. Dazu werden sie beispielsweise 5 h bei einer Temperatur von ca. 470°C an der Luft erhitzt. Dies erfolgt vorteilhaft in einem Umluftofen, bei dem eine Temperaturhomogenität von ± 10°C gewährleistet ist.

Zur Charakterisierung der elektrochemischen Eigenschaften der nach dem erfindungsgemässen Verfahren hergestellten Glaskohlenstoff-Elektroden werden Impedanzmessungen der Doppelschicht herangezogen; dabei ergibt sich folgendes (Messungen in 3,6 M H$_2$SO$_4$). Elektroden aus nach üblichen Verfahren hergestelltem (glattem) Glaskohlenstoff besitzen eine Doppelschichtkapazität von 12 bis 16 μF.cm$^{-2}$. Nach dem erfindungsgemässen Verfahren (unter Verwendung von Stearylmethacrylat) hergestellte Glaskohlenstoff-Elektroden weisen, ohne Aktivierung, bei 1 Hz eine Kapazität von bis zu 1 mF . cm$^{-2}$ auf, was auf eine gewisse Rauhigkeit der Elektrodenoberfläche hindeutet. Nach der Aktivierung ist die Kapazität der erfindungsgemässen Glaskohlenstoff-Elektroden geringen als diejenige von aktivierten Elektroden aus üblichem Glaskohlenstoff, wobei der Unterschied jedoch frequenzabhängig ist. Für üblichen aktivierten Glaskohlenstoff betragen die Werte 330 mF . cm$^{-2}$ bei 1 Hz und 180 mF.cm$^{-2}$ bei 100 Hz, für den erfindungsgemässen aktivierten Glaskohlenstoff 250 mF . cm$^{-2}$ bzw. 160 mF . cm$^{-2}$.

Unter Verwendung von Ethyllaureat (anstelle von Stearylmethacrylat) hergestellte erfindungsgemässe Glaskohlenstoff-Elektroden zeigen folgende Werte: 290 mF . cm$^{-2}$ bei 1 Hz und 165 mF . cm$^{-2}$ bei 100 Hz. Es zeigt sich somit, dass die Einflüsse der oberflächenaktiven Substanzen auf die Doppelschichtkapazität der aktivierten Glaskohlenstoff-Elektroden bei hohen Frequenzen geringer sind als bei niedrigen Frequenzen.

Zur Herstellung der Rohfolie kann beim erfindungsgemässen Verfahren beispielsweise auch folgendermassen vorgegangen werden. 20 g eines käuflichen Phenol-Furanharzes werden mit 2,5 ml einer 20%igen Lösung von P-Toluolsulfonsäure und mit 1 ml Ölsäure (4,5 Masse-%) versetzt, dann wird 5 min lang gerührt und anschliessend 10 min lang entgast (Ölpumpenvakuum). Das Gemisch wird nachfolgend auf eine auf 75°C erwärmte Polypropylenfolie gegossen und mit einer Rakel verteilt. Nach ca. 10 min wird eine flexible, selbsttragende Folie mit einer Dicke von 0,2 bis 0,3 mm erhalten. Diese Folie wird in der bereits beschriebenen Weise zu dünnen, ebenen Glaskohlenstoff-Elektroden weiterverarbeitet.

## Patentansprüche

1. Verfahren zur Herstellung von Glaskohlenstoff-Elektroden durch Härtung und Pyrolyse von duroplastischen carbonisierbaren Harzen, dadurch gekennzeichnet, dass dem Harz 1 bis 10 Masse-% eines pyrolysierbaren organischen Fettalkohol- oder Fettsäurederivates der allgemeinen Formel RCOOR' mit mindestens 14 Kohlenstoffatomen zugesetzt werden und dass das Harz in dünner Schicht auf einer hydrophoben Unterlage ausgebreitet und anschliessend gehärtet und pyrolysiert wird.

2. Verfahren nach Anspruch 1, dadurch gekennzeichnet, dass als Harz Furfurylalkohl verwendet wird.

3. Verfahren nach Anspruch 1, dadurch gekennzeichnet, dass ein Phenol-Furanharz verwendet wird.

4. Verfahren nach einem der Ansprüche 1 bis 3, dadurch gekennzeichnet, dass dem Harz Stearylmethacrylat zugesetzt wird.

5. Verfahren nach einem der Ansprüche 1 bis 3, dadurch gekennzeichnet, dass dem Harz Ethyllaureat oder Ölsäure zugesetzt wird.

6. Verfahren nach einem oder mehreren der Ansprüche 1 bis 5, dadurch gekennzeichnet, dass das Harz mit p-Toluolsulfonsäure gehärtet wird.

7. Verfahren nach einem oder mehreren der Ansprüche 1 bis 6, dadurch gekennzeichnet, dass das Harz bei Temperaturen zwischen 150 und 200°C gehärtet wird.

8. Verfahren nach einem oder mehreren der Ansprüche 1 bis 7, dadurch gekennzeichnet, dass die Pyrolyse bei Temperaturen bis zu 1100°C durchgeführt wird.

9. Verfahren nach einem oder mehreren der Ansprüche 1 bis 8, dadurch gekennzeichnet, dass die Elektroden aktiviert werden.

10. Verwendung der nach dem Verfahren nach einem oder mehreren der Ansprüche 1 bis 9 hergestellten Glaskohlenstoff-Elektroden in elektrochemischen Doppelschichtkondensatoren.

## Claims

1. A process for producing vitreous carbon electrodes by hardening and pyrolysis of duroplastic carbonisable resins, characterised in that 1-10 weight % of a pyrolysable organic fatty alcohol or fatty acid derivative of the general formula RCOOR' with at least 14 carbon atoms in added to the resin and that the resin is spread on a hydrophobic substrate in a thin layer and is then hardened and pyrolysed.

2. A process according to claim 1, characterised in that furfuryl alcohol is used as the resin.

3. A process according to claim 1, characterised in that a phenol furan resin is used.

4. A process according to any one of claims 1 to 3, characterised in that stearyl methacrylate is added to the resin.

5. A process according to any one of claims 1 to 3, characterised in that ethyl laureate or oleic acid is added to the resin.

6. A process according to one or more of claims 1 to 5, characterised in that the resin is hardened with p-toluenesulphonic acid.

7. A process according to one or more of claims 1 to 6, characterised in that the resin is hardened at temperatures between 150 and 200°C.

8. A process according to one or more of claims 1 to 7, characterised in that the pyrolysis is carried out at temperatures of up to 1100°C.

9. A process according to one or more of claims 1 to 8, characterised in that the electrodes are activated.

10. Use of the vitreous carbon electrodes produced by the process according to one or more of claims 1 to 9, in electrochemical double-layer capacitors.

## Revendications

1. Procédé de fabrication d'électrodes en carbone vitreux par durcissement et pyrolyse de résines thermodurcissables susceptibles d'être carbonisées, caractérisé en ce qu'il consiste à ajouter à la résine de 1 à 10 % en poids d'un dérivé d'alcool gras ou d'acide gras organique susceptible d'être pyrolysé, de formule générale RCOOR', ayant au moins 14 atomes de carbone, et à étaler la résine en couche mince sur un support hydrophobe, et ensuite à la durcir et à la pyrolyser.

2. Procédé suivant la revendication 1, caractérisé en ce qu'il consiste à utiliser comme résine de l'alcool furfurylique.

3. Procédé suivant la revendication 1, caractérisé risé en ce qu'il consiste à utiliser une résine phénolfuranne.

4. Procédé suivant l'une des revendications 1 à 3, caractérisé en ce qu'il consiste à ajouter à la résine du méthacrylate de stéaryle.

5. Procédé suivant l'une des revendications 1 à 3, caractérisé en ce qu'il consiste à ajouter à la résine du laurate d'éthyle ou de l'acide oléique.

6. Procédé suivant l'une ou plusieurs des revendications 1 à 5, caractérisé en ce qu'il consiste à durcir la résine par de l'acide p-toluènesulfonique.

7. Procédé suivant l'une ou plusieurs des revendications 1 à 6, caractérisé en ce qu'il consiste à durcir la résine à des températures comprises entre 150 et 200°C.

8. Procédé suivant l'une ou plusieurs des revendications 1 à 7, caractérisé en ce qu'il consiste à effectuer la pyrolyse à des températures allant jusqu'à 1100°C.

9. Procédé suivant l'une ou plusieurs des revendications 1 à 8, caractérisé en ce qu'il consiste à activer les électrodes.

10. Utilisation d'électrodes en carbone vitreux, fabriquées par le procédé suivant l'une ou plusieurs des revendications 1 à 9, dans des condensateurs électrochimiques à couche double.